# EUROPEAN PATENT APPLICATION

(11) **EP 4 778 560 A1**
(43) Date of publication of application: **22.07.2026**
(21) Application number: 23956574.0
(22) Date of filing: 09.11.2023
(51) Int. Cl.: A61M 25/10

(54) **BALLOON DILATION CATHETER**

(30) Priority: 24.10.2023 CN 202311388771
(71) Applicant: Shanghai MicroPort Medical (Group) Co., Ltd., Shanghai 201203 (CN)
(72) Inventor: ZHANG, Haojie, Shanghai 201203 (CN); ZHOU, Qi, Shanghai 201203 (CN); JIA, Jingwei, Shanghai 201203 (CN); CAO, Chunhong, Shanghai 201203 (CN); WANG, Jing, Shanghai 201203 (CN)
(74) Representative: Marks & Clerk LLP
(86) International application number: PCT/CN2023/130626
(87) International publication number: WO 2025/086337

(57) **Abstract**

The present invention provides a balloon dilatation catheter including a catheter body, a blood flow tube and a balloon. The blood flow tube includes a first tubular body and a second tubular body, which is disposed within the first tubular body, an outer wall of a first side of the second tubular body is joined to an inner wall of a first side of the first tubular body, and a blood flow lumen configured for flow of blood is formed between the outer wall of the other side of the second tubular body and the inner wall of the other side of the first tubular body. The balloon is disposed over and attached to an outer wall surface of the first tubular body, an inflation lumen is formed between the balloon and the first tubular body. A distal end of the catheter body is inserted within the second tubular body, a first medium lumen is provided between the distal end of the catheter body and the second tubular body. The first medium lumen is brought into communication with the inflation lumen by a through-part provided at joint of the first and second tubular bodies. The present invention overcomes the blood-supply problem of the distal blood vessel that may arise from expansion of a balloon.

## Description

### TECHNICAL FIELD

The present invention relates to the technical field of medical devices and, in particular, to a balloon dilatation catheter.

### BACKGROUND

Atherosclerosis refers to the thickening and narrowing of the walls of arteries that supply blood to various organs such as the heart, brain, and kidneys. It is a major cause of heart attacks and strokes, and is one of the most common death causes in the world. At present, balloon products have been widely used in interventional treatment of atherosclerosis through percutaneous transluminal angioplasty (PTA). These balloon products may be categorized by their function into, among others, pre-dilation, post-dilation and drug-coated ones.

Pre-dilation balloons are typically used to treat vascular calcification, severe stenosis and chronic total occlusion (OTC) and special lesions. A pre-dilation balloon can be used to dilate a lesion in advance to allow for assessment of its nature and estimation of its treatment complexity, providing guidance for subsequent strategy planning. In addition, such pre-dilation can partially relieve stenosis of a lesion, increasing the success of subsequent passage of a stent through the lesion and reducing damage that may be caused by the lesion to a coating layer on the stent.

Post-dilation balloons are typically used to treat complex, ostial and in-stent lesions by providing post-treatment subsequent to stenting, which ensures adequate stent expansion and apposition, reduces in-stent thrombosis and lowers the incidence of post-stenting restenosis and revascularization.

As novel "implant-free" interventional devices, drug-coated balloons (DCBs) comprise a drug coating layer on the balloon surface, which, as a result of inflating the balloon, is separated from the balloon and adheres to the wall of a blood vessel at a lesion area to deliver the therapeutic effect. This enables the treatment of a severe lesion in a coronary artery, while not leaving a permanent implant within the blood vessel. Thus, not only a series of issues associated with stenting can be circumvented, but retreatment of the diseased blood vessel remains possible. These unique advantages are of great significance.

Such conventional balloons are designed to be inflated until complete occlusion of a blood vessel for improved apposition, enhancement of an intended effect, or more efficient drug transfer. However, in some patients, the distal vessels cannot tolerate ischemia for a long period of time, making it impossible to perform dilation for an adequate duration, which may lead to poor outcomes from the use of a pre- or post-dilation balloon. Moreover, it may disallow maximized transfer of a drug coating layer from a DCB onto a vascular wall and/or its secure adherence to the vascular wall. As a consequence, a lower expected drug concentration may be present at the lesion area, and/or thrombosis of a distal blood vessel may develop. Conclusively, the currently available conventional balloons are still suffering from some limitations during their use in the treatment of various lesions.

It should be noted that the information disclosed in this Background section is merely intended to provide a better understanding of the general context of the present invention and should not be taken as an acknowledgement or any form of admission that the information forms part of the common general knowledge of those skilled in the art.

### SUMMARY OF THE INVENTION

It is an object of the present invention to overcome the blood-supply problem of the distal blood vessel that arises from balloon inflation by presenting a balloon dilatation catheter.

To this end, the present invention provides a balloon dilatation catheter comprising a catheter body, a blood flow tube and a balloon, the blood flow tube extending through both proximal and distal ends of the balloon,
the blood flow tube comprising a first tubular body and a second tubular body, the second tubular body disposed within the first tubular body, wherein an outer wall of a first side of the second tubular body is joined to an inner wall of a first side of the first tubular body, and wherein a blood flow lumen configured for flow of blood is formed between the outer wall of a second side of the second tubular body and the inner wall of a second side of the first tubular body,
wherein the balloon is disposed over and attached to an outer wall of the first tubular body, and wherein an inflation lumen for receiving an inflation medium is provided between the balloon and the first tubular body,
wherein a distal end of the catheter body is inserted within the second tubular body, wherein a first medium lumen configured for passage of the inflation medium therethrough is provided between the distal end of the catheter body and the second tubular body,
wherein a joint of the first and second tubular bodies comprises a through-part configured to bring the first medium lumen into communication with the inflation lumen.

Optionally, the outer wall of the first side of the second tubular body is joined to the inner wall of the first side of the first tubular body by welding, wherein a welding length between the first and second tubular bodies is 2 mm to 6 mm, wherein the through-part comprises at least one through-hole provided at the welding of the first and second tubular bodies.

Optionally, the second and first tubular bodies are of an integral structure, and wherein the through-part comprises an opening slit formed in the joint of the first and second tubular bodies.

Optionally, a proximal end of the first tubular body comprises a proximally tapered first bevel opening, and/or wherein a distal end of the first tubular body comprises a distally tapered second bevel opening.

Optionally, the catheter body comprises an outer tube and an inner tube, wherein a distal end of the outer tube is attached to a proximal end of the second tubular body, wherein a proximal end of the inner tube is located within the distal end of the outer tube, wherein a distal end of the inner tube is located within the second tubular body, wherein the first medium lumen is provided between the inner tube and the second tubular body, wherein a second medium lumen configured for passage of the inflation medium therethrough is provided between the inner and outer tubes, and wherein the second medium lumen is in communication with the first medium lumen.

Optionally, the inner tube comprises a guide wire lumen extending along an axis thereof, wherein one side of the outer tube comprises a guide wire port configured for insertion of a guide wire into the guide wire lumen.

Optionally, an area of the inner tube that is located within the balloon is provided with a radiopaque marker. Additionally or alternatively, a proximal end of the outer tube is provided with a catheter marker strip.

Optionally, the outer tube may comprise a proximal-outer tube and a distal-outer tube, a proximal end of the distal-outer tube joined to a distal end of the proximal-outer tube, a distal end of the distal-outer tube attached to the proximal end of the second tubular body.

Optionally, the balloon dilatation catheter may further comprise a coupling member and a stress dispersion tube, a proximal end of the stress dispersion tube coupled to a distal end of the coupling member, a distal end of the stress dispersion tube coupled to a proximal end of the catheter body.

Optionally, the balloon dilatation catheter may further comprise a head structure, wherein a distal end face of the catheter body and a distal end face of the second tubular body are both attached to a proximal end of the head structure.

Optionally, the blood flow lumen may have a crescent-shaped cross-section.

Optionally, the balloon may have a diameter of 2 mm to 4 mm.

Optionally, the balloon may have a length of 10 mm to 40 mm.

Optionally, the first tubular body may have an outer diameter of 0.7 mm to 1.5 mm.

Optionally, the first tubular body may have a wall thickness of 0.15 mm to 0.25 mm.

Optionally, the first tubular body may have a length of 20 mm to 50 mm.

Optionally, the second tubular body may have an outer diameter of 0.6 mm to 1 mm.

Optionally, the second tubular body may have a length of 25 mm to 55 mm.

Optionally, the balloon may be provided with a drug coating layer on its outer surface.

Optionally, the balloon may be made of nylon, a polyether block amide copolymer or thermoplastic polyurethane.

The balloon dilatation catheter of the present invention offers the following benefits over the prior art:
It includes a catheter body, a blood flow tube and a balloon. The blood flow tube extends through both proximal and distal ends of the balloon. The blood flow tube includes a first tubular body and a second tubular body, which is disposed within the first tubular body, an outer wall of a first side of the second tubular body is joined to an inner wall of a first side of the first tubular body, and a blood flow lumen configured for flow of blood is formed between the outer wall of the other side of the second tubular body and the inner wall of the other side of the first tubular body. The balloon is disposed over and attached to an outer wall of the first tubular body, and an inflation lumen for receiving an inflation medium is provided between the balloon and the first tubular body. A distal end of the catheter body is inserted within the second tubular body, a first medium lumen configured for passage of the inflation medium therethrough is provided between the distal end of the catheter body and the second tubular body. The joint of the first and second tubular bodies comprises a through-part configured to bring the first medium lumen into communication with the inflation lumen. In the present invention, the blood flow tube that extends through the balloon and comprises the blood flow lumen is provided at the distal end of the balloon dilatation catheter to ensure that there is blood flow from the proximal end toward distal tissue both before and after the balloon is expanded, preventing distal ischemia that may otherwise result from the expansion of the balloon. In this way, it solves the problem associated with conventional balloons, which completely occlude the vessel for a period of time during pre-dilation, post-dilation, or drug delivery, thereby reducing the therapeutic effect in patients who have poor tolerance to distal vascular ischemia. In addition, according to the present invention, multiple separate lumens are formed by multiple nesting tubes. This not only allows the lumens to provide their functions, but can also minimize a crossing diameter of the balloon dilatation catheter.

### BRIEF DESCRIPTION OF THE DRAWINGS

Fig. 1 shows a schematic overall view of a balloon dilatation catheter according to an embodiment of the present invention.
Fig. 2 shows a schematic diagram of a proximal portion of the balloon dilatation catheter according to the embodiment of the present invention.
Fig. 3 shows a schematic plan view of a distal portion of the balloon dilatation catheter according to the embodiment of the present invention.
Fig. 4 shows a schematic perspective view of the distal portion of the balloon dilatation catheter according to the embodiment of the present invention.
Fig. 5 shows a cross-sectional view of the distal portion of the balloon dilatation catheter according to the embodiment of the present invention, which is in an expanded configuration.
Fig. 6 shows a cross-sectional view of the proximal portion of the balloon dilatation catheter according to the embodiment of the present invention, which is in a collapsed configuration.
Fig. 7 shows blood flow in a conventional balloon dilatation catheter in an inflated configuration.
Fig. 8 shows blood flow in the balloon dilatation catheter according to the embodiment of the present invention in an inflated configuration.
Fig. 9 shows a schematic diagram of a blood flow tube in a balloon dilatation catheter according to another embodiment.

### List of Reference Numerals

catheter body-100; outer tube-110; guide wire port-111; proximal-outer tube-112; distal-outer tube-113; inner tube-120; guide wire lumen-121; radiopaque marker-140; catheter marker strip-150;
blood flow tube-200; first tubular body-210; first bevel opening-211; second bevel opening-212; second tubular body-220; blood flow lumen-230; through-part-240;
balloon-300;
inflation lumen-410; first medium lumen-420;
coupling member-510; stress dispersion tube-520; head structure-530.

### DETAILED DESCRIPTION

Balloon dilatation catheters according to specific embodiments of the present invention disclosed herein will be described below in greater detail with reference to the accompanying drawings. From the following description, advantages and features of the invention will become more apparent. Note that the figures are provided in a very simplified form not necessarily drawn to exact scale for the only purpose of helping to explain the disclosed embodiments in a more convenient and clearer way. In order that the objects, features and advantages of the present invention can be more apparent and readily understood, reference is to be made to the accompanying drawings. It would be recognized that architectural, proportional, dimensional and other details in the figures are presented only for the purpose of facilitating, in conjunction with the disclosure herein, the understanding and reading of those familiar with the art, rather than being intended to limit conditions under which the present invention can be implemented. Therefore, any and all architectural modifications, proportional variations or dimensional changes that achieve the same or similar benefits and objects as the present invention are considered to fall within the scope of the teachings herein. The specific design features of the invention as disclosed herein, including, for example, specific dimensions, orientations, locations and shapes, will be determined in part by the particular intended application and use environment. Additionally, in the embodiments described below, same reference numerals may be sometimes used to refer to the same or functionally same elements throughout different figures, while description thereof may not be repeated. In this specification, the same reference numerals and letters refer to the same items in the figures, and thus once an item is defined in one figure, it may not be discussed for following figures. Further, if a method is described herein as comprising a series of steps, the order of these steps as presented herein is not necessarily the only order in which they can be performed, and some of the stated steps may be omitted and/or other steps not described herein may be added to the method.

It is noted that relational terms such as "first," "second," and the like may be used herein solely to distinguish one entity or action from another entity or action, without necessarily requiring or implying any actual such relationship or order between such entities or actions, or denoting or implying relative importance of them, or implicitly indicating the number of them. Moreover, the terms "comprising", "including" and any variations thereof are intended to cover a non-exclusive inclusion, such that a process, method, article, or apparatus that comprises a list of elements is not necessarily limited to those elements, but may include other elements not expressly listed or inherent to such process, method, article, or apparatus. An element preceded by "comprises... a" does not, without more constraints, preclude the existence of additional identical elements in the process, method, article, or apparatus that comprises the element. As used herein, the singular forms "a", "an" and "the" include plural referents, and the term "or" is generally employed in the sense of "and/or", "a number of" is generally employed in the sense of "at least one", "at least two" is generally employed in the sense of "two or more", and "plurality" is generally employed in the sense of "at least two".

It will be understood that terms such as "central", "longitudinal", "transverse", "length", "width", "thickness", "upper", "lower", "front", "rear", "left", "right", "vertical", "horizontal", "top", "bottom", "inner", "outer", "axial", "radial", "circumferential", etc. may be used herein to describe directional or positional relationships based on the orientations shown in the figures. They are intended merely to facilitate and simplify the explanation of the invention and do not indicate or imply that the stated components or elements have to comprise, or be constructed or operated in, particular orientations. Therefore, they are not to be construed as limiting the invention. As used herein, unless otherwise clearly specified or defined, the terms "mounted", "coupled", "connected", "secured" and variants thereof should be interpreted in a broad sense. For instance, a connection may be a permanent, detachable or integral connection, or a mechanical or electrical connection, or a direct or indirect connection with one or more intervening media, or an internal communication or interaction between two elements. Those of ordinary skill in the art can understand the specific meanings of the above-mentioned terms herein, depending on their context. As used herein, unless otherwise clearly specified or defined, when a first feature is described as being "above" or "below" a second feature, it may be either in direct contact with the second feature, or in indirect contact with one or more intervening media being present therebetween. When a first feature is referred to as being "on", "above" or "on top of" a second feature, it may be right or obliquely on, above or on top of the second feature, or simply located at a higher level than the second feature. When a first feature is referred to as being "under", "below" or "at bottom of" a second feature, it may be right or obliquely under, below or at bottom of the second feature, or simply located at a lower level than the second feature.

In principle, the present invention seeks to solve the blood-supply problem that arises from balloon inflation in a distal blood vessel by presenting a balloon dilatation catheter.

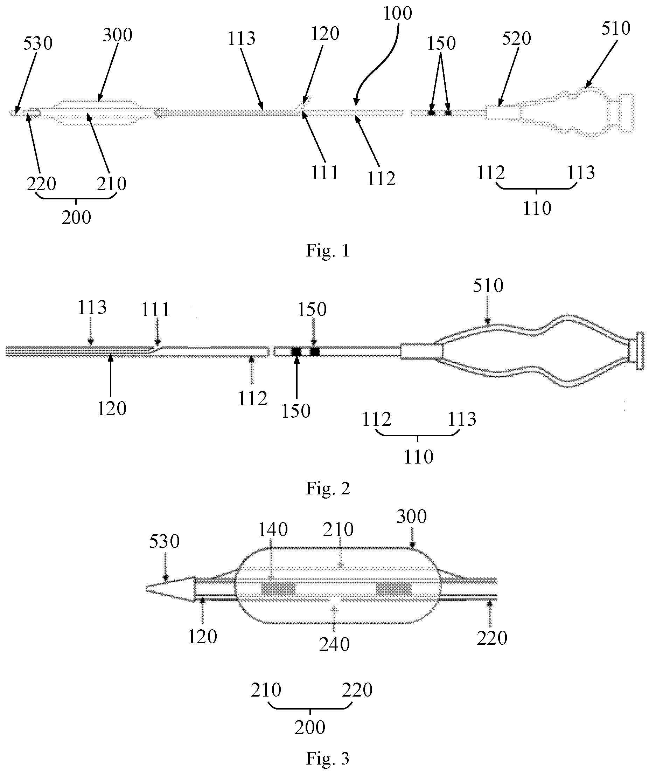

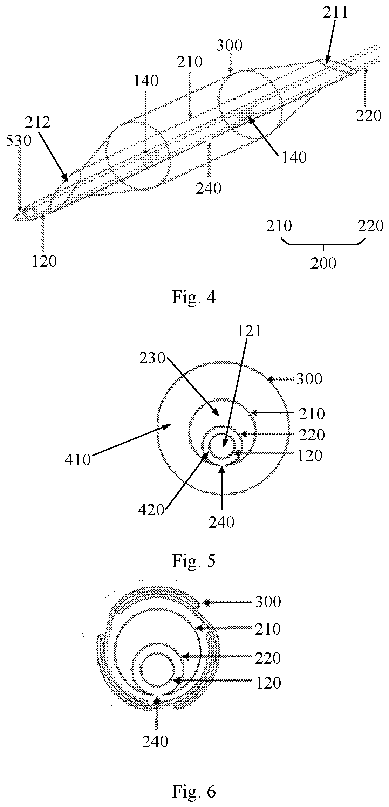

Reference is made to Figs. 1 to 6. Fig. 1 shows a schematic overall view of a balloon dilatation catheter according to an embodiment of the present invention. Fig. 2 shows a schematic diagram of a proximal portion of the balloon dilatation catheter according to the embodiment of the present invention. Fig. 3 shows a schematic plan view of a distal portion of the balloon dilatation catheter according to the embodiment of the present invention. Fig. 4 shows a schematic perspective view of the distal portion of the balloon dilatation catheter according to the embodiment of the present invention. Fig. 5 shows a cross-sectional view of the distal portion of the balloon dilatation catheter according to the embodiment of the present invention, which is in an expanded configuration. Fig. 6 shows a cross-sectional view of the proximal portion of the balloon dilatation catheter according to the embodiment of the present invention, which is in a collapsed configuration. As shown in Figs. 1 to 6, a balloon dilatation catheter provided by the present invention includes a catheter body 100, a blood flow tube 200 and a balloon 300. The blood flow tube 200 extends through both proximal and distal ends of the balloon 300. The blood flow tube 200 includes a first tubular body 210 and a second tubular body 220. The second tubular body 220 is disposed within the first tubular body 210, with outer wall of one side of the second tubular body 220 being joined to an inner wall of one side of the first tubular body 210. Thus, a blood flow lumen 230 configured for flow of blood therethrough is formed between the outer wall of the other side of the second tubular body 220 and the inner wall of the other side of the first tubular body 210. The balloon 300 is disposed over and attached to an outer wall of the first tubular body 210, and an inflation lumen 410 for containing an inflation medium therein is formed between the balloon 300 and the first tubular body 210. A distal end of the catheter body 100 is inserted within the second tubular body 220, and a first medium lumen 420 configured for passage of the inflation medium therethrough is formed between the distal end of the catheter body 100 and the second tubular body 220. The first medium lumen 420 is brought into communication with the inflation lumen 410 by a through-part 240 provided in the joined portions of the first tubular body 210 and the second tubular body 220. According to the present invention, the blood flow tube 200 that extends through the balloon 300 and comprises the blood flow lumen 230 is provided in a distal portion of the balloon dilatation catheter to ensure that there is blood flow from the proximal end toward distal tissue both before and after the balloon 300 is expanded (or inflated), preventing distal ischemia that may otherwise result from the expansion of the balloon 300. This solves the problem of conventional balloons 300 completely blocking the blood vessel for a period of time during pre-dilation, post-dilation, or drug delivery, which reduces the therapeutic effect for patients with poor distal ischemia tolerance. In addition, by adopting nesting tubes to form multiple independent lumens, the present invention ensures the functionality of each lumen (including the first medium lumen 420, the inflation lumen 410, the blood flow lumen 230, and the second medium lumen described below) while minimizing the crossing profile of the balloon dilation catheter. It should be noted, and will be appreciated by those skilled in the art, that the blood flow lumen 230 is isolated from the first medium lumen 420 and the inflation lumen 410. That is, the blood flow lumen 230 does not communicate with either of the first medium lumen 420 and the inflation lumen 410.

Specifically, the balloon dilatation catheter comprises a collapsed configuration and an expanded configuration (i.e., an inflated configuration). The balloon 300 may be inflated or expanded by introducing a gas or liquid (e.g., a contrast fluid) therein from an external source, thereby inflating or expanding the balloon to allow the balloon dilatation catheter in the expanded configuration, as shown in Fig. 5. The balloon dilatation catheter will remain in the collapsed configuration before the balloon 300 is inflated or expanded, as shown in Fig. 6. In addition, the blood flow tube 200 will not be crushed or deformed as a consequence of the balloon 300 being folded or crimped, ensuring that there is blood flow from the proximal end toward distal tissue both before and after the balloon 300 is expanded (or inflated).

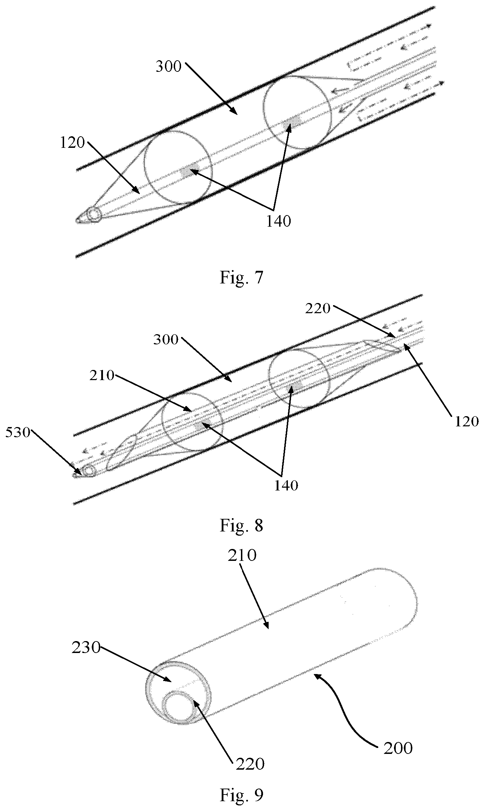

Reference is further made to Figs. 7 and 8. Fig. 7 shows blood flow in a conventional balloon dilatation catheter in an inflated configuration. Fig. 8 shows blood flow in the balloon dilatation catheter according to the embodiment of the present invention that is in the inflated configuration. As shown in Fig. 7, when blood flow from the proximal side reaches the inflated balloon 300 in the conventional balloon dilatation catheter, it will be prevented from further movement toward distal tissue due to close apposition of the balloon 300 against the vascular wall. As shown in Fig. 8, when the balloon dilatation catheter of the present invention is in the inflated configuration, the blood flow lumen 230 formed between the first tubular body 210 and the second tubular body 220 provides a channel allowing for blood flow from the proximal side to reach distal tissue. It should be noted, and will be appreciated by those skilled in the art, that the dashed arrows in Figs. 7 and 8 indicate the direction of blood flow.

Continued reference is made to Figs. 5 and 6. As shown in Figs. 5 and 6, in some exemplary embodiments, the blood flow lumen 230 has a crescent-shaped cross-section (taken perpendicular to its axis). This crescent-like shape of the blood flow lumen 230 allows blood to flow into the blood flow lumen 230 at a greater angle along the catheter's circumference, thereby maximizing entry of blood into the blood flow lumen 230 while avoiding distal blood flow obstruction.

Continued reference is made to Fig. 4. As shown in Fig. 4, in some exemplary embodiments, a proximal end of the first tubular body 210 comprises a proximally tapered first bevel opening 211, and a distal end of the first tubular body 210 comprises a distally tapered second bevel opening 212. By providing the first bevel opening 211 at the proximal end of the first tubular body 210 and the second bevel opening 212 at the distal end of the first tubular body 210, it can not only provide enlarged blood inlet area, but also enable blood to partially enter the blood flow lumen 230 in the form of turbulent flow, allowing for increased blood flow, as well as less resistance to advancement of the balloon dilatation catheter, and hence easier delivery of the balloon dilatation catheter to a lesion site within a blood vessel. It should be noted, and will be appreciated by those skilled in the art, that in some alternative embodiments, only the second bevel opening 212 may be provided at the distal end of the first tubular body 210 to reduce resistance to advancement of the balloon dilatation catheter, or only the first bevel opening 211 may be provided at the proximal end of the first tubular body 210 to increase the blood flow.

In particular, the first bevel opening 211 and the second bevel opening 212 may be provided in the form of chamfers at the proximal and distal ends of the first tubular body 210, respectively. Of course, as will be appreciated by those skilled in the art, the present invention is not so limited, as the first bevel opening 211 and the second bevel opening 212 may also be provided otherwise.

Additionally, the first bevel opening 211 may be tapered at an angle of 45° to 60°, and the second bevel opening 212 may be tapered at an angle of 45° to 60°. Selecting the taper angles from those ranges can not only prevent a blood vessel from being scratched at side walls at the first bevel opening 211 and the second bevel opening 212, but can also provide even larger blood entrance area, further increasing entry of blood.

In some exemplary embodiments, the balloon 300 is made of nylon, polyether block amide (Pebax) or thermoplastic polyurethane (TPU). The balloon 300 made of nylon, Pebax, TPU or a similar material enables the balloon dilatation catheter of the present invention to be suitably used in various applications such as pre-dilation, post-dilation and drug delivery.

In some exemplary embodiments, the balloon 300 has a diameter of 2 mm to 4 mm and a length of 10 mm to 40 mm. Such dimensions make the balloon dilatation catheter of the present invention more suitable for use in various applications such as pre-dilation, post-dilation and drug delivery.

In some exemplary embodiments, the first tubular body 210 has an outer diameter of 0.7 mm to 1.5 mm, a wall thickness of 0.15 mm to 0.25 mm and a length of 20 mm to 50 mm. Such dimensions can not only avoid the first tubular body 210 from being crushed or deformed as a consequence of the balloon 300 being folded or crimped and thereby ensure that there is blood flow from the proximal end toward distal tissue both before and after the balloon 300 is expanded (or inflated), but can also ensure that the first tubular body 210 will not be crushed or deformed within an operating pressure range of balloon 300 and thus allow smooth flow of blood therethrough.

Additionally, the first tubular body 210 may be made of nylon or Pebax. The first tubular body 210 made of nylon or Pebax will exhibit sufficient strength, which ensures that the first tubular body 210 will not be crushed or deformed during folding or crimping of the balloon 300 and hence that there is blood flow from the proximal end toward distal tissue both before and after the balloon 300 is expanded (or inflated).

In some exemplary embodiments, the second tubular body 220 has an outer diameter of 0.6 mm to 1 mm and a length of 25 mm to 55 mm. Such dimensions can ensure that the crescent-shaped blood flow lumen 230 is formed between the first tubular body 210 and the second tubular body 220, allowing blood to flow into the blood flow lumen 230 at a greater angle along the catheter's circumference.

Additionally, the second tubular body 220 may be made of nylon or Pebax. The second tubular body 220 made of nylon or Pebax will exhibit sufficient strength, which ensures that the blood flow tube 200 will not be crushed or deformed during folding or crimping of the balloon 300 and hence that there is blood flow from the proximal end toward distal tissue both before and after the balloon 300 is expanded (or inflated).

In some exemplary embodiments, the balloon 300 is provided with a drug coating layer over its outer surface. The drug coating layer over the outer surface of the balloon 300 can be adequately released into the diseased blood vessel, thereby effectively reducing the probability of intimal hyperplasia and minimizing vascular elastic recoil.

In particular, the drug may be coated over the outer surface of the balloon 300 by electrostatic spraying, ultrasonic spraying, crystal growth, thereby forming a drug coating layer over the outer surface of the balloon 300. The coating layer may be made up simply of the drug, or of a mixture of the drug and a carrier matrix. Examples of the drug may include, but are not limited to, at least one of rapamycin, rapamycin derivatives, paclitaxel and paclitaxel derivatives. Examples of the carrier matrix may include, but are not limited to, at least one of iopromide, urea, polyoxyethylene and polylactic acid.

In some exemplary embodiments, the outer wall of one side of the second tubular body 220 is welded to the inner wall of one side of the first tubular body 210, and a welding length between the second tubular body 220 and the first tubular body 210 ranges from 2 mm to 6 mm, and the through-part 240 includes at least one through-hole formed in the welded portion of the first tubular body 210 and the second tubular body 220. Therefore, by configuring the first tubular body 210 and the second tubular body 220 as separate structures and connecting the outer wall of the second tubular body 220 on one side to the inner wall of the first tubular body 210 on one side by welding to form the blood flow tube 200 having a crescent-shaped blood vessel lumen, the production cost of the balloon dilation catheter provided by the present invention can be reduced. In addition, by setting the welding length between the second tubular body 220 and the first tubular body 210 to 2 mm ~ 6 mm, it is possible to prevent the flexibility (pushability) of the balloon dilation catheter in the blood flow tube 200 segment from being affected due to an excessively long welding length. Further, by configuring at least one through-hole in the welded portions of the first tubular body 210 and the second tubular body 220 to form the through-part 240 that brings the first medium lumen 420 in communication with the inflation lumen 410 can effectively prevent gas leakage.

It should be noted, and will be appreciated by those skilled in the art, that in some alternative embodiments, the outer wall of one side of the second tubular body 220 may be joined to the inner wall of one side of the first tubular body 210 by adhesive bonding or otherwise. For more details in this regard, reference is made to related techniques known to those skilled in the art, and further description thereof is omitted herein.

Further, one or more through-holes may be obtained at the welding of the first tubular body 210 and the second tubular body 220 by piercing with a fine needle. Preferably, the through-part 240 includes a single through-hole, to reduce the process risk points. It should be noted, and will be appreciated by those skilled in the art, that the piercing area should not be beyond a boundary of the weld seam, in order to prevent air leakage.

Continued reference is made to Fig. 1. As shown in Fig. 1, in some exemplary embodiments, the catheter body 100 includes an outer tube 110 and an inner tube 120. A distal end of the outer tube 110 is attached to a proximal end of the second tubular body 220. A proximal end of the inner tube 120 is located within the outer tube 110 at the distal end thereof, and a distal end of the inner tube 120 is disposed within the second tubular body 220. The first medium lumen 420 is formed between the inner tube 120 and the second tubular body 220, and a second medium lumen configured for passage of the inflation medium therethrough is formed between the inner tube 120 and the outer tube 110, the second medium lumen is in communication with the first medium lumen 420. Therefore, by configuring the catheter body 100 as a dual-layer structure comprising an outer tube 110 and an inner tube 120, not only can the balloon dilation catheter provided by the present invention achieve good delivery performance, but also external gas or liquid can be delivered to the first medium lumen 420 through the second medium lumen formed by the gap between the inner tube 120 and the outer tube 110, and then delivered to the inflation lumen 410 through the through-part 240, thereby inflating the whole balloon 300. It should be noted, and will be appreciated by those skilled in the art, that the blood flow lumen 230 is isolated from the second medium lumen. That is, the blood flow lumen 230 does not communicate with the second medium lumen.

Continued reference is made to Figs. 1, 2 and 5. As shown in Figs. 1, 2 and 5, in some exemplary embodiments, the inner tube 120 comprises a guide wire lumen 121 extending along an axis thereof. On one side of the outer tube 110, a guide wire port 111 is provided for the guidewire to enter the guide wire lumen 121. A guide wire may be threaded through the guide wire port 111 and the guide wire lumen 121 so that the balloon dilatation catheter can be moved along the guide wire to a lesion area. In particular, the balloon 300 may be moved in the collapsed configuration along the guide wire to the lesion area, and the inflation medium (e.g., a contrast fluid or gas) may be then introduced through the second medium lumen, the first medium lumen 420 and the through-part 240 into the inflation lumen 410 to inflate the entire balloon 300 until it tightly adheres to the wall of a blood vessel containing the lesion. When the balloon 300 is kept at a constant inflation pressure the blood flow is still allowed from the proximal end towards distal tissue through the reserved blood flow lumen 230.

Continued reference is made to Fig. 1. As shown in Fig. 1, in some exemplary embodiments, the proximal end of the inner tube 120 protrudes out of the guide wire port 111. This facilitates threading of the guide wire.

Continued reference is made to Figs. 3 and 4. As shown in Figs. 3 and 4, in some exemplary embodiments, a section of the inner tube 120 located within the balloon 300 is provided with a radiopaque marker 140, which allows the balloon 300 to be located by radiography. In particular, the radiopaque marker 140 may be integrally formed with the inner tube 120. Alternatively, the radiopaque marker 140 can be integrally formed with the inner tube 120, that is, a portion of the inner tube 120 may be made of a radiopaque material. Still alternatively, the radiopaque marker 140 may be fabricated, for example, as a radiopaque ring, separately from the inner tube 120 and then embedded in or on the inner tube 120.

Additionally, as shown in Figs. 3 and 4, two radiopaque markers 140 may be provided, one corresponds to the proximal end of the balloon 300 and the other corresponds to the distal end of the balloon 300. Thus, the balloon 300 can be even more easily located by radiography through configuration of two radiopaque markers 140.

Continued reference is made to Figs. 1 and 2. As shown in Figs. 1 and 2, in some exemplary embodiments, the proximal end of the outer tube 110 is provided with catheter marker strip 150. Therefore, by providing a catheter marker strip 150 at the proximal end of the outer tube 110, it can be used to indicate the length of the balloon dilation catheter inserted into the patient's body.

Continued reference is made to Figs. 1 and 2. As shown in Figs. 1 and 2, in some exemplary embodiments, the outer tube 110 includes a proximal-outer tube 112 and a distal-outer tube 113. A proximal end of the distal-outer tube 113 is joined to a distal end of the proximal-outer tube 112, and a distal end of the distal-outer tube 113 is attached to the proximal end of the second tubular body 220. By configuring the outer tube 110 to include the proximal-outer tube 112 and the distal-outer tube 113 structure imparts both good pushability and good kink resistance to the entire balloon dilatation catheter of the present invention, which enable its smooth passage through small tortuous vessels and facilitate its retraction. Thus, multiple dilation cycles can be performed, and the time required for a surgical procedure is reduced. In addition, the proximal-outer tube 112 may be stiffer than the distal-outer tube 113. This can additionally enhance the pushability of the balloon dilatation catheter of the present invention.

Continued reference is made to Figs. 1 and 2. As shown in Figs. 1 and 2, in some exemplary embodiments, the balloon dilatation catheter further includes a coupling member 510 and a stress dispersion tube 520. A proximal end of the stress dispersion tube 520 is coupled to a distal end of the coupling member 510, and a distal end of the stress dispersion tube 520 is coupled to a proximal end of the catheter body 100 (more precisely, of the proximal-outer tube 112). By using a stress dispersion tube 520 to connect the coupling member 510 and the catheter body 100, the strength of the connection between the coupling member 510 and the catheter body 100 can be enhanced, which can effectively prevent the balloon dilatation catheter of the present invention from breakage during its use.

Continued reference is made to Figs. 1 and 3. As shown in Figs. 1 and 3, in some exemplary embodiments, the balloon dilatation catheter further includes a head structure 530. A distal end face of the catheter body 100 and a distal end face of the second tubular body 220 are both attached to a proximal end of the head structure 530. The head structure 530 is provided at a distal end of the balloon dilatation catheter to pass through a lesion tissue.

Additionally, as shown in Figs. 1 and 3, in some exemplary embodiments, the head structure 530 has a diameter gradually decreasing from its proximal end to distal end. With this arrangement, the head structure 530 can more easily pass through a lesion tissue.

Fabrication of the balloon dilatation catheter according to this embodiment is detailed below.

First of all, a tube made of Pebax or other material, which has an outer diameter of 0.6 mm to 1 mm, such as 0.7 mm, and a length of 25 mm to 55 mm, such as 35 mm, is selected as the second tubular body 220, and another tube made of nylon 12 or a other material, which has an outer diameter of 0.7 mm to 1.5 mm, such as 1.25 mm, a length of 20 mm to 50 mm, such as 30 mm, and a wall thickness of 0.15 mm to 0.25 mm, such as 0.2 mm, is then selected as the first tubular body 210.

Subsequently, the inner wall of one side of the first tubular body 210 is welded to the outer wall of one side of the second tubular body 220, a welding length is 2 mm to 6 mm, such as 4 mm. This step forms the first nesting, thereby forming the blood flow lumen 230.

A fine needle is used to pierce the welded portions of the first tubular body 210 and the second tubular body 220 to obtain one or more through-holes, so as to form the through-part 240.

Afterwards, the balloon 300 is attached to both ends of the outer wall of the first tubular body 210. The balloon 300 is made of Pebax or other material and has an outer diameter of 2 mm to 4 mm, such as 3 mm, and a length of 10 mm to 40 mm, such as 20 mm. This step forms the second nesting, thereby forming the inflation lumen 410.

The proximal end of the second tubular body 220 is then attached to the distal-outer tube 113, and the distal end of the second tubular body 220 is secured to the inner tube 120 through the head structure 530, the proximal end of the inner tube 120 provides an entrance for the guide wire port 111. This step forms the third nesting, the interior of the inner tube 120 is the guide wire lumen 121, and the first medium lumen 420 is formed between the inner tube 120 and the second tubular body 220. Moreover, the second medium lumen is formed between the inner tube 120 and the outer tube 110 (including the proximal-outer tube 112 and the distal-outer tube 113), and the first medium lumen 420 is in communication with the second medium lumen.

Reference is further made to Fig. 9, which shows a schematic diagram of a blood flow tube 200 in a balloon dilatation catheter according to another embodiment of the present invention. As shown in Fig. 9, the balloon dilatation catheter of this embodiment differs from that of the previous embodiment in that the second tubular body 220 and the first tubular body 210 are an integral structure formed with each other, and the though-part 240 comprises an opening slit at the joint of the second tubular body 220 and the first tubular body 210, in this embodiment. Forming the second tubular body 220 and the first tubular body 210 as a one-piece integral structure can alleviate the problems of reduced catheter compliance and increased process inconsistency that may arise from multiple welding operations. The opening slit is provided at the joint of the first tubular body 210 and the second tubular body 220 to from the through-part 240. In this way, the enlarged through-part 240 may be provided, enabling faster inflation and deflation of the balloon 300.

It should be noted that although Fig. 9 does not show the first bevel opening 211 and the second bevel opening 212 of the first tubular body 210, as will be appreciated by those skilled in the art, the first bevel opening 211 and the second bevel opening 212 may be provided as chamfers at the proximal and distal ends of the first tubular body 210, respectively. Of course, as will be appreciated by those skilled in the art, the first bevel opening 211 and the second bevel opening 212 may also be provided in any other suitable form, without departing from the scope of the invention.

Fabrication of the balloon dilatation catheter according to this embodiment is detailed below.

At first, a dual-lumen tube including both the first tubular body 210 and the second tubular body 220 is co-extruded, which is made of nylon 12 or other material and has an outer diameter of 0.7 mm to 1.5 mm, such as 1.25 mm, and a length of 20 mm to 50 mm, such as 30 mm. A crescent-shaped cavity formed between an outer circumference of the second tubular body 220 and an inner circumference of the first tubular body 210 provides the blood flow lumen 230, and the inner lumen of the second tubular body 220 (i.e., the circular lumen of the dual-lumen tube) and the inner tube 120 subsequently passing therethrough form the first medium lumen 420.

On one side of an outer wall of the dual-lumen tube (in particular, at a side where the first tubular body 210 and the second tubular body 220 are joined), an opening slit is made by laser scribing to obtain an opening slit, thereby an opening silt to form the through-part 240, so as to bring the first medium lumen 420 into communication with the subsequently inflation lumen 410 but not with the crescent-shaped blood flow lumen 230 to prevent air leakage.

Subsequently, the balloon 300 is welded to both ends of the outer wall of the dual-lumen tube. The balloon 300 is made of Pebax or other material and has an outer diameter of 2 mm to 4 mm, such as 3 mm, and a length of 10 mm to 40 mm, such as 20 mm. This step forms the inflation lumen 410.

After that, the proximal end of the second tubular body 220 (the circular lumen) of the dual-lumen tube is coupled to the distal-outer tube 113, and the distal end of the second tubular body 220 is secured to the inner tube 120 through the head structure 530, the proximal end of the inner tube 120 provides an entrance for the guide wire port 111. Consequently, the interior of the inner tube 120 provides the guide wire lumen 121, and the first medium lumen 420 is formed between the inner tube 120 and the second tubular body 220 of the dual-lumen tube (the circular lumen). Moreover, the second medium lumen is formed between the inner tube 120 and the outer tube 110 (including the proximal-outer tube 112 and the distal-outer tube 113), the first medium lumen 420 is in communication with the second medium lumen.

At last, a drug coating layer may be formed on a surface of the balloon 300 by applying everolimus to the surface through ultrasonic spraying, and the balloon 300 may be then folded or crimped, thereby obtaining the balloon dilatation catheter.

In summary, the balloon dilatation catheter of the present invention offers the following benefits over the prior art:
(1) The blood flow tube 200 that extends through the balloon 300 and comprises the blood flow lumen 230 is provided at the distal end of the balloon dilatation catheter to ensure that there is blood flow from the proximal end toward distal tissue both before and after the balloon 300 is expanded, preventing distal ischemia that may otherwise result from the expansion of the balloon 300. Thereby, the problem is solved that existing conventional balloons 300, when used for pre-dilation, post-dilation, or drug delivery, cause complete occlusion of the blood vessel for a period of time, thus reducing the therapeutic effect for some patients with poor tolerance to distal ischemia.
(2) Multiple separate lumens are formed by multiple nesting tubes. This not only allows the lumens to provide their functions, but can also minimize a crossing diameter of the balloon dilatation catheter.
(3) The crescent-like shape of the blood flow lumen 230 allows blood to flow into the blood flow lumen 230 at a greater angle along the catheter's circumference. This maximizes entry of blood into the blood flow lumen 230 while avoiding distal blood flow obstruction.

It should be noted that reference throughout this specification to "one embodiment", "some embodiments", "an example", "a specific example" "some examples" or the like means that a particular feature, structure, material, or characteristic described in connection with the embodiments or examples is included in at least one embodiment or example of the invention. Thus, the appearances of those phrases in various places throughout this specification are not necessarily referring to the same embodiment or example of the invention. Furthermore, the particular features, structures, materials, or characteristics may be combined in any suitable manner in any one or more embodiments. Further, should there be no contradiction, one of ordinary skill in the art can combine the various embodiments, examples and features thereof described herein in any combination.

It should also be noted that the description presented above is merely that of a few preferred embodiments of the present invention and is not intended to limit the scope thereof in any sense. Any and all changes and modifications made by those of ordinary skill in the art in light of the above teachings fall within the scope as defined in the appended claims. Apparently, those skilled in the art can make various modifications and variations to the present invention without departing from the spirit and scope thereof. Accordingly, the invention is intended to embrace all such modifications and variations if they fall within the scope of the appended claims and equivalents thereof.

## Claims

1. A balloon dilatation catheter, comprising a catheter body, a blood flow tube and a balloon, wherein the blood flow tube extends through a proximal end and a distal end of the balloon,
wherein the blood flow tube comprises a first tubular body and a second tubular body, wherein the second tubular body is disposed within the first tubular body, wherein an outer wall of a first side of the second tubular body is joined to an inner wall of a first side of the first tubular body, and wherein a blood flow lumen configured for flow of blood is formed between the outer wall of a second side of the second tubular body and the inner wall of a second side of the first tubular body,
wherein the balloon is disposed over and attached to an outer wall of the first tubular body, and wherein an inflation lumen for receiving an inflation medium is provided between the balloon and the first tubular body,
wherein a distal end of the catheter body is inserted within the second tubular body, wherein a first medium lumen configured for passage of the inflation medium therethrough is provided between the distal end of the catheter body and the second tubular body,
wherein a joint of the first and second tubular bodies comprises a through-part configured to bring the first medium lumen into communication with the inflation lumen.

2. The balloon dilatation catheter according to claim 1, wherein the outer wall of the first side of the second tubular body is joined to the inner wall of the first side of the first tubular body by welding, wherein a welding length between the first and second tubular bodies is 2 mm to 6 mm, wherein the through-part comprises at least one through-hole provided at the welding of the first and second tubular bodies.

3. The balloon dilatation catheter according to claim 1, wherein the second and first tubular bodies are of an integral structure, and wherein the through-part comprises an opening slit formed in the joint of the first and second tubular bodies.

4. The balloon dilatation catheter according to claim 1, wherein a proximal end of the first tubular body comprises a proximally tapered first bevel opening, and/or wherein a distal end of the first tubular body comprises a distally tapered second bevel opening.

5. The balloon dilatation catheter according to claim 1, wherein the catheter body comprises an outer tube and an inner tube, wherein a distal end of the outer tube is attached to a proximal end of the second tubular body, wherein a proximal end of the inner tube is located within the distal end of the outer tube, wherein a distal end of the inner tube is located within the second tubular body, wherein the first medium lumen is provided between the inner tube and the second tubular body, wherein a second medium lumen configured for passage of the inflation medium therethrough is provided between the inner and outer tubes, and wherein the second medium lumen is in communication with the first medium lumen.

6. The balloon dilatation catheter according to claim 5, wherein the inner tube comprises a guide wire lumen extending along an axis thereof, wherein one side of the outer tube comprises a guide wire port configured for insertion of a guide wire into the guide wire lumen.

7. The balloon dilatation catheter according to claim 5, wherein an area of the inner tube that is located within the balloon is provided with a radiopaque marker, and/or wherein a proximal end of the outer tube is provided with a catheter marker strip.

8. The balloon dilatation catheter according to claim 5, wherein the outer tube comprises a proximal-outer tube and a distal-outer tube, wherein a proximal end of the distal-outer tube is joined to a distal end of the proximal-outer tube, and wherein a distal end of the distal-outer tube is attached to the proximal end of the second tubular body.

9. The balloon dilatation catheter according to claim 1, further comprising a head structure, wherein each of a distal end face of the catheter body and a distal end face of the second tubular body is attached to a proximal end of the head structure.

10. The balloon dilatation catheter according to claim 1, further comprising at least one of:
wherein a cross-section of the blood flow lumen comprises a crescent shape;
wherein a diameter of the balloon is 2 mm to 4 mm;
wherein a length of the balloon is 10 mm to 40 mm;
wherein an outer diameter of the first tubular body is 0.7 mm to 1.5 mm;
wherein a wall thickness of the first tubular body is 0.15 mm to 0.25 mm;
wherein a length of the first tubular body is 20 mm to 50 mm;
wherein an outer diameter of the second tubular body is 0.6 mm to 1 mm;
wherein a length of the second tubular body is 25 mm to 55 mm;
wherein an outer surface of the balloon is provided with a drug coating layer; and
wherein the balloon is made of nylon, a polyether block amide copolymer or thermoplastic polyurethane.
